# EUROPEAN PATENT APPLICATION

(11) **EP 3 950 931 A1**
(43) Date of publication of application: **09.02.2022**
(21) Application number: 20782451.7
(22) Date of filing: 27.03.2020
(51) Int. Cl.: C12N 5/071, C12Q 1/02

(54) **CULTURED TISSUE AND METHOD FOR PRODUCING SAME**

(30) Priority: 29.03.2019 JP 2019066417
(71) Applicant: NAGASAKI UNIVERSITY, Nagasaki 852-8521 (JP); Eisai R&D Management Co., Ltd., Tokyo 112-8088 (JP)
(72) Inventor: SAKAI Yusuke, Nagasaki-shi, Nagasaki 852-8521 (JP); EGUCHI Susumu, Nagasaki-shi, Nagasaki 852-8521 (JP); ADACHI Tomohiko, Nagasaki-shi, Nagasaki 852-8521 (JP); HUANG Yu, Nagasaki-shi, Nagasaki 852-8521 (JP); SHINKYO Raku, Tsukuba-shi, Ibaraki 300-2635 (JP); SAGARA Saki, Tsukuba-shi, Ibaraki 300-2635 (JP); ONOZATO Daichi, Tsukuba-shi, Ibaraki 300-2635 (JP)
(74) Representative: Hoffmann Eitle
(86) International application number: PCT/JP2020/014222
(87) International publication number: WO 2020/203850

(57) **Abstract**

A cultured tissue, comprising: glandular cells; glandular cavities formed from the glandular cells; and ducts formed from epithelial cells, wherein the glandular cavities and the ducts are functionally connected *ex vivo.*

## Description

### Technical Field

The present invention relates to a cultured tissue and a method for producing the same.

### Background Art

The development of biomaterials such as artificial organs and graft materials by tissue engineering has been required for application to medical treatment, and research mainly for forming tissue structure *in vitro* has been conducted in recent years. A tissue model imitating an *in vivo* tissue can be used for clarifying the basic mechanism of the tissue, and can be used for evaluation at an *in vitro* tissue level before the evaluation of the pharmacokinetics or the safety of a compound which is an active ingredient candidate of a remedy by animal experiments. Therefore, various cultured tissues have been reported as tissue models until now (for example, Patent Literatures 1 and 2).

Glands are tissues which perform secretion, and include endocrine glands which send out secretions to blood vessels or lymph vessels directly and exocrine glands which release secretions through ducts to the body surface or body cavities. An exocrine gland has glandular cells which secrete a secretion, glandular cavities which store a secretion secreted from the glandular cells temporarily, and ducts which transport a secretion from glandular cavities to the body surface or body cavities. A glandular cavity is a lumen surrounded and formed by glandular cells, and a secretion from glandular cells is first secreted to a glandular cavity. Glandular cavities and ducts are joined, and form channels for secreting a secretion from glandular cells to the body surface or body cavities.

### Citation List

### Patent Literature

Patent Literature 1 JP 2018-029611
Patent Literature 2 JP 2018-166512

### Summary of Invention

### Technical Problem

Even though cells were cultured *in vitro,* a glandular tissue model was constructed, it was difficult to connect glandular cavities and ducts functionally in the past. When glandular cavities and ducts are not functionally connected, a secretion secreted from glandular cells does not flow through ducts, remains, and is retained in glandular cavities, and may lead to the necrosis of the glandular cells depending on circumstances.

Then, an object of the present invention is to provide a cultured tissue in which glandular cavities and ducts are functionally connected, and a secretion secreted from glandular cells to the glandular cavities flows into the ducts.

### Solution to Problem

The present inventors have earnestly investigated repeatedly, found a method for producing a cultured tissue in which glandular cavities and ducts are functionally connected, and completed the present invention.

That is, one embodiment of the present invention is as follows.
[1] A cultured tissue, comprising:
   glandular cells; glandular cavities formed from the glandular cells; and
   ducts formed from epithelial cells,
   wherein the glandular cavities and the ducts are functionally connected *ex vivo.*
[2] The cultured tissue according to [1], wherein the cultured tissue is an exocrine gland.
[3] The cultured tissue according to [1] or [2], wherein the glandular cells are hepatocytes, and the epithelial cells are biliary epithelial cells.
[4] The cultured tissue according to [1] or [2], wherein the glandular cells are pancreatic acinar cells, and the epithelial cells are pancreatic ductal epithelial cells.
[5] The cultured tissue according to any one of [1] to [4], wherein the epithelial cells are epithelial cells induced from progenitor cells.
[6] The cultured tissue according to [5], wherein the progenitor cells are progenitor cells reprogrammed from mature cells.
[7] The cultured tissue according to any one of [1] to [6], wherein the glandular cells are primary cells.
[8] The cultured tissue according to [3], wherein the glandular cells are primary hepatocytes, and the biliary epithelial cells are biliary epithelial cells induced from liver progenitor cells, and here, the liver progenitor cells are liver progenitor cells reprogrammed from hepatocytes.
[9] The cultured tissue according to any one of [1] to [8], wherein the epithelial cells are rodent cells.
[10] The cultured tissue according to any one of [1] to [9], wherein the glandular cells are human cells.
[11] The cultured tissue according to any one of [1] to [3] and [5] to [10], wherein the glandular cells are human hepatocytes.
[12] The cultured tissue according to any one of [1] to [3] and [5] to [11], wherein the epithelial cells are rodent biliary epithelial cells.
[13] A method for producing a cultured tissue, comprising:
   culturing epithelial cells to form ducts from the epithelial cells and
   coculturing the ducts and glandular cells so that the ducts and the glandular cells are in contact.
[14] The method according to [13], wherein the coculture is performed by two-dimensional culture.
[15] The method according to [13] or [14], wherein the glandular cells are hepatocytes, and the epithelial cells are biliary epithelial cells.
[16] The method according to [13] or [14], wherein the glandular cells are pancreatic acinar cells, and the epithelial cells are pancreatic ductal epithelial cells.
[17] The method according to any one of [13] to [16], further comprising:
   inducing differentiation of progenitor cells to the epithelial cells.
[18] The production method according to [17], further comprising:
   reprogramming mature cells to the progenitor cells before the induction of differentiation of the progenitor cells to the epithelial cells.
[19] The method according to any one of [13] to [18], wherein the glandular cells are primary cells.
[20] The method according to any one of [13] to [19], wherein the epithelial cells are rodent cells.
[21] The method according to any one of [13] to [20], wherein the glandular cells are human cells.
[22] The method according to any one of [13] to [15] and [17] to [21], wherein the glandular cells are human hepatocytes.
[23] The production method according to any one of [13] to [15] and [17] to [22], wherein the epithelial cells are rodent biliary epithelial cells.
[24] A method for evaluating a test substance using the cultured tissue according to any one of [1] to [12].
[25] A kit for evaluating a test substance, comprising: the cultured tissue according to any one of [1] to [12].

### Advantageous Effects of Invention

According to the present invention, a cultured tissue in which glandular cavities and ducts are functionally connected, and a secretion secreted from glandular cells to the glandular cavities flows into the ducts can be provided.

### Brief Description of The Drawings

[Figure 1A] Figure 1A is a conceptual diagram describing a procedure for inducing biliary epithelial cells from rat CLiPs (rCLiPs) and forming bile ducts.
[Figure 1B] Figure 1B is phase-contrast microscope images showing the appearance of induction from the rCLiPs to the biliary epithelial cells and bile duct formation, and is images 1 day, 6 days, and 12 days after the inoculation of the rCLiPs on mouse embryonic fibroblasts (MEFs) from the left.
[Figure 2A] Figure 2A is a conceptual diagram describing a procedure for coculturing bile ducts formed from rCLiPs and rat hepatocytes.
[Figure 2B] Figure 2B is phase-contrast microscope images showing the appearance of the coculture of the bile ducts formed from the rCLiPs and the rat hepatocytes. The images are images 1 day and 3 days after the coculture is started from the left.
[Figure 3A] Figure 3A is photographs showing the accumulation of bile acid in bile ducts cocultured with hepatocytes. The upper figure is phase-contrast microscope images, the lower figure is fluorescence microscope images, and the images are photographs 2 hours, 24 hours, and 72 hours after cholyl-lysyl-fluorescein (CLF) is added to the medium from the left, respectively.
[Figure 3B] Figure 3B is a phase-contrast microscope image (left figure) and a fluorescence microscope image (right figure) 2 hours after CLF is added to medium containing only bile ducts and not cocultured with hepatocytes.
[Figure 4A] Figure 4A is a conceptual diagram describing a procedure for inducing biliary epithelial cells from rCLiPs and forming bile ducts.
[Figure 4B] Figure 4B is phase-contrast microscope images showing the appearance of the induction from the rCLiPs to the biliary epithelial cells and bile duct formation, and is images 1 day, 6 days, and 12 days after the rCLiPs are inoculated on mouse embryonic fibroblasts (MEFs) from the left.
[Figure 5A] Figure 5A is a conceptual diagram describing a procedure for coculturing bile ducts formed from rCLiPs and human hepatocytes.
[Figure 5B] Figure 5B is phase-contrast microscope images showing the appearance of the coculture of the bile ducts formed from the rCLiPs and the human hepatocytes. The images are images 1 day and 3 days after the coculture is started from the left.
[Figure 6A] Figure 6A is photographs showing the accumulation of bile acid in bile ducts cocultured with human hepatocytes. The upper figure is phase-contrast microscope images, the lower figure is fluorescence microscope images, and the images are photographs directly, 4 hours, and 24 hours after the cells are incubated in Hanks' balanced salt solution (HBSS) containing CLF and then washed with HBSS not containing CLF from the left, respectively.
[Figure 6B] Figure 6B is a photograph showing bile ducts not cocultured with human hepatocytes. The upper figure is phase-contrast microscope images, the lower figure is fluorescence microscope images, and the images are photographs directly, 4 hours, and 24 hours after the cells are incubated in HBSS containing CLF and then washed with HBSS not containing CLF from the left, respectively.

### Description of Embodiments

### [Cultured tissue]

The present invention provides a cultured tissue, comprising: glandular cells; glandular cavities formed from the glandular cells; and ducts formed from epithelial cells, wherein the glandular cavities and the ducts are functionally connected *ex vivo.* The "cultured tissue" refers to a cell assembly which can function as a living tissue of a human or an animal except a human, and is artificially cultured *ex vivo* herein.

### [Exocrine gland]

In one embodiment of the present invention, it is preferable that a cultured tissue be exocrine glands. The "exocrine glands" refer to glands in which glandular cells discharge a secretion thereof through ducts to the body surface or the insides of body cavities such as the intestinal tract and the trachea herein. When the cultured tissue is exocrine glands, blood vessels are excluded from the "ducts". Examples of the exocrine glands include digestive glands, sweat glands, sebaceous glands, and mammary glands. Examples of the digestive glands include the liver, the pancreas, salivary glands, gastric glands, and intestinal glands. In one embodiment of the present invention, it is preferable that the exocrine glands be the liver or the pancreas, and it is more preferable that the exocrine glands be the liver.

### [Glandular cell, glandular cavity, and duct]

The "glandular cells" refer to cells which secrete a secretion herein. The "glandular cavities" refer to cavities formed from the glandular cells herein. The "ducts" refer to pipes which are formed from the epithelial cells, and transport secretions herein. In one embodiment of the present invention, the glandular cells may be cells which secrete a secretion, and can form the glandular cavities, and the epithelial cells may be epithelial cells having the capability to form lumens. The glandular cells and the epithelial cells can be suitably selected depending on the cultured tissue to be constructed.

"Connecting functionally" and "functional connection" mean that the glandular cavities and the ducts are linked so that the secretion flow into the ducts from the glandular cavities herein. In such connection, the glandular cavities and the ducts may be connected directly and continuously, or the glandular cavities and the ducts may be connected continuously through intercellular substances such as the extracellular matrix. For example, such connection also includes the state in which a labeled secretion or an analogue thereof is incorporated into the glandular cells, it is confirmed whether the labeled secretion or an analogue thereof secreted from the glandular cells can be detected from the ducts or not, and the labeled secretion or the analogue thereof can be confirmed from the ducts.

"The glandular cavities and the ducts are functionally connected *ex vivo*" means that the functional connection between the glandular cavities and the ducts is performed *ex vivo.* Therefore, a cultured tissue obtained by culturing a glandular tissue extracted from the living body as it stands is not included in the present invention.

In one embodiment of the present invention, examples of the glandular cells contained in the cultured tissue of the present invention include primary cells, established cell lines, and glandular cells induced from pluripotent stem cells (for example, iPS cells and ES cells). The "primary cells" refer to cells collected from the living body and cultured, and not established herein. Therefore, the "primary cells" herein can also include cells subcultured from cells collected from the living body for several passages. When the glandular cells are primary cells, the glandular cells maintain actual function of *in vivo* cells better than the established cell lines, and the cultured tissue to be constructed can therefore be a tissue model more similar to that *in vivo.* Glandular cells induced from pluripotent stem cells can be used instead of the primary cells. Such pluripotent stem cells are, for example, iPS cells. In another embodiment, when the cultured tissue to be constructed is a tissue model of a glandular tissue of a patient contracting cancer, glandular cells of an established cancer cell line may be used.

### [Liver tissue]

In one embodiment of the present invention, the cultured tissue is a liver tissue. The liver is an internal organ which functions as the center of metabolic reactions of the living body. The liver plays various roles such as the metabolism of sugars, proteins, and lipids obtained from foods; the synthesis of plasma proteins; the decomposition of red blood cells; the decomposition of alcohols, drugs, toxins, and waste matter; and the secretion of bile.

When the cultured tissue is a liver tissue, the glandular cells are hepatocytes (also called liver parenchymal cells). Hepatocytes are main cells which constitute the liver, and are glandular cells which secrete bile, which is a digestive juice. Hepatocytes play roles such as metabolism and biosynthesis conducted in the liver besides the secretion of bile. A single hepatocyte is a polyhedron of around 25 to 30 µm, and have surfaces facing capillary vessels called sinusoids and surfaces on which hepatocytes are adhered. Hepatocytes are in a raw along a sinusoid, and form palisade tissues called hepatic cords *in vivo.* Hepatocytes form glandular cavities called biliary canaliculi between adjacent hepatocytes in hepatic cords. Bile produced in hepatocytes is secreted in biliary canaliculi. In one embodiment of the present invention, the hepatocytes should account for many of cell groups thereof, and liver non-parenchymal cells may be contained, or the hepatocytes may be constituted from only the hepatocytes. For example, although many of the cell groups of primary hepatocytes are constituted from hepatocytes, the primary hepatocytes can also contain liver non-parenchymal cells.

Biliary epithelial cells play the role of adjusting the flow rate of bile, which hepatocytes secrete, the ion concentration, the pH, and the like. Biliary epithelial cells gather and form a tubular structure. This pipe is a duct which transports bile to the gallbladder and the duodenum, namely the bile duct.

In one embodiment, the cultured tissue of the present invention is a liver tissue wherein the glandular cells are hepatocytes, the secretion is bile, the glandular cavities are biliary canaliculi, the epithelial cells are biliary epithelial cells, and the ducts are bile ducts. According to the liver tissue related to one embodiment of the present invention, bile is secreted from the hepatocytes to the biliary canaliculi, the biliary canaliculi and the bile duct are functionally connected, bile therefore flows into the ducts without being retained in the biliary canaliculi. Such a liver tissue can be used for evaluating the drug efficacy, the toxicity, and the pharmacokinetics, and the like of the test substance in the liver.

It is preferable that the biliary epithelial cells forming the bile ducts be biliary epithelial cells induced from liver progenitor cells. Here, the "progenitor cells" also include cells in a stage during differentiation from the pluripotent stem cells to specific mature cells and more undifferentiated cells induced from mature cells herein. Accordingly, "liver progenitor cells" may be cells in a stage during differentiation from liver stem cells to hepatocytes, or may be more undifferentiated cells induced from hepatocytes, which are mature cells, herein. The "mature cells" means somatic cells, which are not derived from germ cells or reproductive cells, and examples include hepatocytes constituting the liver herein. It is preferable to induce the liver progenitor cells to the biliary epithelial cells in two-dimensional culture. When the bile ducts are formed in two-dimensional culture, it is easy to perform the coculture mentioned below in which the hepatocytes and the bile ducts are in contact.

It is preferable that the liver progenitor cells induced to the biliary epithelial cells be the liver progenitor cells to which the hepatocytes, which are mature cells, are reprogrammed. Here, the "reprogramming" means a process in which mature cells are induced to cells of which the developmental potency is higher than that of the mature cells, in other words, a process in which mature cells are returned to a more undifferentiated state, herein. In the cultured tissue according to one embodiment of the present invention, the hepatocytes can be reprogrammed to the liver progenitor cells by culturing hepatocytes under predetermined conditions and a predetermined method. The liver progenitor cells to which the hepatocytes are reprogrammed is well-known as CLiPs (chemically induced liver progenitor cells), and such reprogramming conditions and such a reprogramming method are described in detail in Takeshi Katsuda et al., Conversion of Terminally Committed Hepatocytes to Culturable Bipotent Progenitor Cells with Regenerative Capacity, Cell Stem Cell, January 5, 2017, 20th volume, p.1-15 (hereinafter called "Reference 1").

Since it is difficult for the liver progenitor cells to be collected and isolated from the living body, and identified, liver progenitor cells in which the purity is high can be obtained more efficiently using the liver progenitor cells to which the hepatocytes are reprogrammed than by collection and isolation from the living body, and identification.

As mentioned above, according to one embodiment of the present invention, a cultured tissue in which the biliary canaliculi and the bile ducts are functionally connected, and bile which the hepatocytes secrete into the biliary canaliculi flows from the biliary canaliculi to the bile ducts is provided.

### [Pancreas tissue]

In another embodiment, the cultured tissue of the present invention is pancreas tissue. While the pancreas is an endocrine gland which secretes blood sugar-regulating hormones such as insulin and glucagon in the blood, the pancreas secretes pancreatic juice containing various digestive enzymes which decompose sugars, proteins, and lipids obtained from foods as an exocrine gland. Pancreatic acinar cells are main cells which account for 90% or more of the whole pancreas, and are glandular cells which secrete pancreatic juice. A dozen or so pancreatic acinar cells form glandular cavities called acini, and pancreatic acinar cells secrete pancreatic juice into the acini.

A plurality of pancreatic ductal epithelial cells (also called pancreatic ductal cells) gathers and forms a duct called the pancreatic duct, and the pancreatic duct transports pancreatic juice, which is secreted by acini, to the duodenum. Pancreatic duct epithelial cells themselves secrete water containing hydrogen carbonate ions. The hydrogen carbonate ion solution secreted from pancreatic ductal cells constitutes a part of pancreatic juice with pancreatic juice from the acini, the part of pancreatic juice passes the pancreatic duct, and flows toward the duodenum.

One embodiment of the cultured tissue of the present invention is a pancreas tissue in which the glandular cells are pancreatic acinar cells, the secretion is pancreatic juice, the glandular cavities are acini, the epithelial cells are pancreatic ductal epithelial cells, and the ducts are pancreatic ducts. Since the acini and the pancreatic ducts are functionally connected, and pancreatic juice secreted from the pancreatic acinar cells to the acini flows into the pancreatic ducts without retention in the acini, the pancreas tissue of the present invention can be used as a tissue model of the pancreas as an exocrine gland.

### [Other tissues]

In other embodiments, the cultured tissue of the present invention may be salivary glands such as parotids, submaxillary glands, or sublingual glands; gastric glands such as fundic glands, cardiac glands, or pyloric glands; intestinal glands such as duodenal glands or Lieberkuhn's glands; sweat glands such as eccrine glands or apocrine glands; or sebaceous glands or mammary glands. The combination of the glandular cells and the ducts constituting the glandular tissue is well-known to those skilled in the art, and can be suitably selected depending on the cultured tissue to be constructed.

### [Use of cultured tissue]

In one embodiment, the cultured tissue of the present invention can be used for *ex vivo* evaluation of the drug efficacy, the toxicity, and the pharmacokinetics such as absorption, distribution, metabolism, and excretion of a test substance in the glandular tissue. That is, the present invention also provides a method for evaluating such a test substance using the cultured tissue according to one embodiment of the present invention.

In one embodiment, the present invention also provides a kit for evaluating such a test substance *ex vivo.* According to such a kit, the test substance can be easily evaluated *ex vivo.* The evaluation kit comprises the cultured tissue according to one embodiment of the present invention, and may further comprise at least one of medium; positive control compounds wherein the drug efficacy, the toxicity, and/or the pharmacokinetics in the cultured tissue are known; reagents used for predetermined inspection; materials; tools and an apparatus; and an instruction manual.

In another embodiment, the cultured tissue of the present invention can be used as a use for performing basic research and the evaluation of the *in vivo* glandular tissue *ex vivo.*

In another embodiment, the cultured tissue of the present invention can be used for evaluating the behavior of cancer cells in the glandular tissue, and the metabolism and the excretion of an anticancer agent.

In another embodiment, the cultured tissue of the present invention can be used as a graft for transplant surgery.

In one embodiment of the present invention, the glandular cells and the epithelial cells may be derived from the same species of animal or different species of animals. In an object of the *ex vivo* evaluation of the test substance, the glandular cells may be cells of a human or an animal other than a human (for example, a rodent such as a mouse or a rat). In one embodiment, the glandular cells are human glandular cells, and examples of the human glandular cells include human hepatocytes. It is preferable that the epithelial cells be epithelial cells easily induced to ducts. Examples of such epithelial cells include epithelial cells of a rodent such as a mouse or a rat. In an embodiment, the cultured tissue according to one embodiment of the present invention, human glandular cells and rodent epithelial cells are used. When the cultured tissue according to one embodiment of the present invention is for a graft, it is preferable that both glandular cells and epithelial cells be cells derived from the same species of animal as the recipient animal.

### [Method for producing cultured tissue]

A method for producing a cultured tissue of the present invention comprises culturing epithelial cells to form ducts and coculturing the ducts and the glandular cells so that the ducts and the glandular cells are in contact. In each step, the above-mentioned epithelial cells and glandular cells can be used.

The step of culturing the epithelial cells to form the ducts can be performed by various well-known methods. For example, when the epithelial cells are inoculated on a scaffold material such as an extracellular matrix or feeder cells suitable for culturing epithelial cells and cultured with suitable medium added, the epithelial cells have polarity, and tubular structures, namely ducts, are therefore formed by the epithelial cells so that the apical side of the cells faces lumens, the side surface side faces adjacent epithelial cells, and the bottom side faces the scaffold material.

Examples of the extracellular matrix which can be used as a scaffold material include collagen gel, fibronectin, and laminin besides Matrigel, which is used in the Examples mentioned below, and, for example, human mesenchymal stem cells, human fibroblasts, and the like can be used as the feeder cells besides mouse embryonic fibroblasts (MEFs), which are used in the Examples. The above-mentioned extracellular matrices and feeder cells can be used alone or in combination two or more types.

Examples of mediums which can be used for forming ducts include mTeSR(TM) 1 medium, hepatocyte culture medium, biliary epithelial cell culture medium, islet cell culture medium, small intestine cell culture medium, Dulbecco's modified Eagle's medium (DMEM), Eagle's minimum essential medium (EMEM), Eagle's minimum essential medium α modified type (α-MEM), Glasgow's minimum essential medium (GMEM), Nutrient Mixture F-12 Ham (Ham's F-12), Dulbecco's modified Eagle's medium/Nutrient Mixture F-12 Ham (D-MEM/Ham's F-12), Iscove's modified Dulbecco's medium (IMDM), RPMI-1640 medium, Dulbecco's phosphate buffered saline (D-PBS), and Hanks' balanced salt solution (HBSS) besides BIM-1 medium and BIM-2 medium, which are used in the Examples, and these media can be used alone or in combination of two or more, and can be modified suitably. The medium can be exchanged or changed suitably for a fresh medium or a medium having a new composition, depending on the culture period and the cell number.

Those skilled in the art can set culture temperature and culture time for culturing epithelial cells suitably depending on the types of epithelial cells to be cultured and ducts to be formed. For example, when bile ducts are formed from biliary epithelial cells derived from a human or a rodent, the culture temperature can be 30 to 39°C or 36 to 38°C. The culture time can be, for example, 4 to 720 hours, 120 to 336 hours, or 168 to 288 hours.

The density of epithelial cells to be inoculated can be, for example, 5 × 10³ to 5 × 10⁶ cells/cm² or 5 × 10⁴ to 5 × 10⁵ cells/cm².

The step of coculturing the formed ducts and the glandular cells is performed subsequently to the above-mentioned step or simultaneously with the above-mentioned step so that the ducts and the glandular cells are in contact. "The ducts and the glandular cells are in contact" include both of an aspect in which the ducts and the glandular cells are in direct contact and an aspect in which the ducts and the glandular cells are in contact through intercellular substances such as an extracellular matrix herein. The glandular cavities and the ducts become in a structure in which the glandular cavities and the ducts are functionally connected, and the secretion secreted from the glandular cells to the glandular cavities comes to flow into the ducts by coculturing the formed ducts and the glandular cells with the formed ducts and the glandular cells in contact. Although the reason why the glandular cavities and the ducts are thus functionally connected is not clear, it is considered that signal transmission between the cells with the epithelial cells forming the ducts and the glandular cells forming the glandular cavities in contact is required for the functional connection between the glandular cavities and the ducts.

As long as the ducts and the glandular cells are in contact, the culture method may be two-dimensional culture such as plate culture or three-dimensional culture, and it is preferable to coculture the ducts and the glandular cells by two-dimensional culture. When the coculture is performed by two-dimensional culture, the ducts and the glandular cells are easily in contact, and a structure in which the glandular cavities and the ducts are functionally connected is therefore formed easily.

The ducts and the glandular cells can be cocultured in two-dimensional culture, for example, by first forming the ducts from the epithelial cells on a two-dimensional culture apparatus such as a plate for cell culture, inoculating the glandular cells directly on the formed ducts so as to be in contact with the ducts, and culturing the ducts and the glandular cells as they are. Therefore, the step of forming the ducts and the step of coculturing the ducts and the glandular cells can be successively or simultaneously performed on the same two-dimensional culture apparatus. As long as the ducts are formed, the timing when coculture with the glandular cells is started may be any timing, but can be, for example, 4 to 720 hours, 120 to 336 hours, or 168 to 288 hours after duct induction.

In the case of two-dimensional culture, the density of the glandular cells to be inoculated can be, for example, 3 × 10³ to 6 × 10⁵ cells/cm², 1 × 10⁴ to 1 × 10⁵ cells/cm², or 3 × 10⁴ to 6 × 10⁴ cells/cm².

The temperature for coculturing the ducts and the glandular cells can be, for example, 30 to 39°C or 36 to 38°C. The Time for coculture can be, for example, 4 to 720 hours, 24 to 168 hours, or 48 to 72 hours.

The cell number of the glandular cells in coculture can be, for example, 0.001 to 1000 times, 0.01 to 100 times, or 0.05 to 10 times the cell number of the epithelial cells before induction to the ducts. However, those skilled in the art can set the culture temperature, the culture time, the cell number ratio of the glandular cells to the epithelial cells suitably depending on the types of the ducts and the glandular cells to be cocultured.

Those skilled in the art can select the medium in which the ducts and the glandular cells are cocultured suitably depending on the types of the ducts and the glandular cells to be cocultured. For example, media mentioned as the above-mentioned medium which can be used for forming the ducts can be used alone or in combination of two or more besides BIM-1 medium, which is used in the Examples, and can be modified suitably. The medium can be exchanged or changed suitably for a fresh medium or a medium having a new composition depending on the culture period and the cell number.

The glandular cells to be inoculated on the ducts may be single isolated cells or cell assemblies in which the glandular cells have already assembled and formed glandular cavity structures at the time of the inoculation. Even though the glandular cells are single isolated cells at the time of the inoculation, the glandular cells can assemble and form the glandular cavities during the coculture. The glandular cells forming the glandular cavities contact with the ducts, and the glandular cavities and the ducts are functionally connected. When the glandular cells to be inoculated on the ducts are human hepatocytes, the form of the human hepatocytes is not particularly limited, and examples also include human hepatocytes prepared in the form of a suspension. In such a case, the suspension is inoculated on the ducts. The method for preparing the human hepatocytes can be performed by various well-known methods.

A method according to another embodiment of the present invention may further comprise inducing differentiation of progenitor cells to the above-mentioned epithelial cells forming the ducts. For example, when the cultured tissue to be produced is a liver tissue, such a step is a step of inducing differentiation of liver progenitor cells to biliary epithelial cells. It is preferable to perform not only the step of coculture but also the step of inducing differentiation of the progenitor cells to the epithelial cells and the step of forming the ducts by two-dimensional culture. When these steps are performed by two-dimensional culture, the step of differentiation-inducing the progenitor cells to the epithelial cells, the step of forming the ducts, and the step of coculture can be successively or simultaneously performed on the same two-dimensional culture apparatus. The ducts and the glandular cells can be efficiently contacted in the coculture step, and it is easy to connect the glandular cavities and the ducts more functionally in the cultured tissue to be produced by performing these steps on the same two-dimensional culture apparatus.

A method according to another embodiment of the present invention may further comprise reprogramming mature cells to the above-mentioned progenitor cells before inducing differentiation of the above-mentioned progenitor cells to the epithelial cells. A well-known method can be used for reprogramming the mature cells to the progenitor cells. The mature cells to be reprogrammed may be primary cells or established cells. For example, when the target cultured tissue is a liver tissue, and the epithelial cells are biliary epithelial cells, the above-mentioned mature cells to be reprogrammed may be hepatocytes, and the progenitor cells may be liver progenitor cells. The step of reprogramming the hepatocytes to the liver progenitor cells can be performed, for example, in accordance with the conditions and the method described in the above-mentioned Reference 1. When the target cultured tissue is a liver tissue, both mature cells and glandular cells to be reprogrammed can be hepatocytes.

It can be confirmed by various methods using labeled compounds that the glandular cavities and the ducts are functionally connected in the cultured tissue produced by the above-mentioned method. For example, a compound obtaining by labeling a compound the behavior of which in the glandular cavities and the ducts is known and which are objects to be confirmed can be used. The functional connection between the glandular cavities and the ducts can be specifically confirmed by confirming whether the labeled secretion or the analogue thereof is incorporated into the glandular cells, and the labeled secretion or the analogue thereof secreted from the glandular cells can be detected from the ducts. For example, when the glandular cells are hepatocytes, the glandular cavities are biliary canaliculi, and the ducts are bile ducts, cholyl-lysyl-fluorescein (CLF), which is a bile acid analog labeled fluorescently, can be used as such a labeled secretion or the analogue thereof. When CLF is incorporated into the hepatocytes cocultured with the bile ducts, and the fluorescence of CLF is detected from the bile ducts, it can be confirmed that CLF is secreted from the hepatocytes to the biliary canaliculi, and the secreted CLF moves to the bile ducts, namely that the biliary canaliculi and the bile ducts are functionally connected. As the labeled compound, a compound labeled with a radioactive isotope can be used besides the fluorescent label. It can also be confirmed using an unlabeled compound the behavior of which in the glandular cavities and the ducts is known that the glandular cavities and the ducts are functionally connected. In this case, the functional connection between the glandular cavities and the ducts can be confirmed by measuring the compound in the medium or the cells using a high-performance liquid chromatograph (HPLC) and a mass spectrometer. In a kit according to one embodiment of the present invention, the labeled or unlabeled compound, the behavior of which is known in the glandular cavities and the ducts to be examined, can be optionally included as a positive control.

The test substance in the glandular tissue can be evaluated in the same method using the test substance labeled or not labeled with fluorescence or a radioactive isotope. Those skilled in the art can select the detection and the measurement of a test substance from well-known methods such as the detection of fluorescence, the detection of radioactive rays, and the combination of HPLC and mass spectrometry suitably depending on the test substance, whether it is labeled or not, and the type of the label.

### EXAMPLES

Hereinafter, the present invention will be further described using the Examples. However, the present invention is not interpreted within limitation to the following Examples.

Example 1. Production of cultured tissue using rat hepatocytes

### <Preparation of rat CLiPs>

Rat hepatocytes were reprogrammed to liver progenitor cells in accordance with the method described in the above-mentioned Reference 1.

The liver extracted from a 7-week-old male Wistar rat was specifically perfused by the two-step collagenase perfusion method to separate primary hepatocytes. The primary hepatocytes were inoculated on a collagen-coated dish at a density of 1 × 10⁴ cells/cm². As medium, the following SHM (small hepatocyte culture medium) + YAC medium were used.

SHM + YAC medium: medium containing basic medium (DMEM/F12 medium containing NaHCO₃ and L-glutamine) and further containing the following: 5 mM HEPES, 30 mg/L L-proline, 0.05% BSA, a 10 ng/mL epidermal growth factor (EGF), insulin-transfenin-sodium selenite (ITS)-X supplement (produced by Thermo Fisher Scientific K.K.), 10⁻⁷ M dexamethasone, 10 mM nicotinamide, 1 mM ascorbic acid-2 phosphoric acid (Asc2P), 100 units/mL penicillin, 100 µg/mL streptomycin, 10 µM Y-27632, 0.5 µM A-83-01, and 3 µM CHIR99021.

The medium was exchanged once every 2 days, and culture was performed for 12 to 14 days until the cells are confluent, and rCLiPs were produced.

The cells were treated with TrypLE (TM) Express (produced by Thermo Fisher Scientific K.K.) for 10 minutes and dissociated, and the produced rCLiPs were subcultured.

### <Induction from rCLiPs to biliary epithelial cells and bile duct formation>

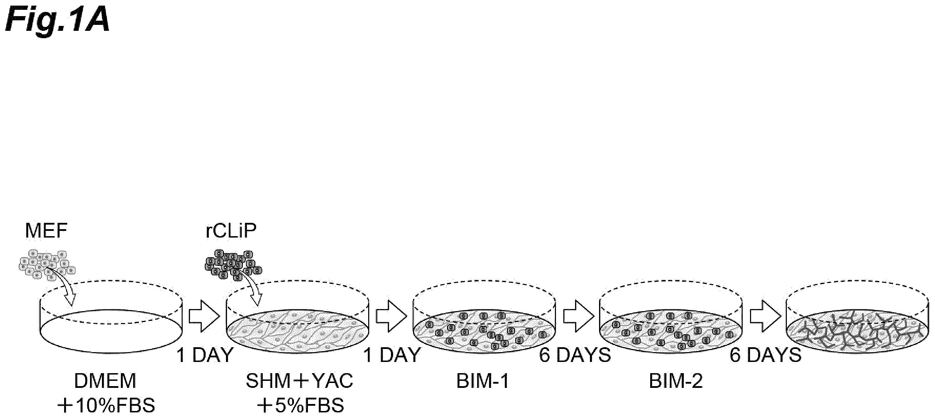

As illustrated in Figure 1A below, biliary epithelial cells were induced from the rCLiPs, and bile ducts were formed.

Mouse embryonic fibroblasts (MEFs) treated with mitomycin were inoculated on a collagen-coated dish at a density of 5.3 × 10⁴ cells/cm² and cultured for 1 day. Medium containing a low glucose DMEM medium and 10% fetal bovine serum (FBS) was used for the medium at the time of inoculating the MEFs.

One day after the inoculation of the MEFs, the subcultured rCLiPs were inoculated on the MEFs at a density of 1.3 × 10⁵ cells/cm². Medium containing the above-mentioned SHM + YAC medium and 5% FBS was used for the medium at the time of inoculating the rCLiPs.

One day after rCLiP inoculation, the medium was exchanged for the following BIM (bile duct-inducing medium)-1 medium, culture was then performed for 6 days to obtain biliary epithelial cells. The BIM-1 medium was newly exchanged once every 2 days during the culture.

BIM-1 medium: medium containing mTeSR(TM) 1 (produced by STEMCELL (TM) Technologies Inc.), 10 µM Y-27632, 0.5 µM A-83-01, and 3 µM CHIR99021.

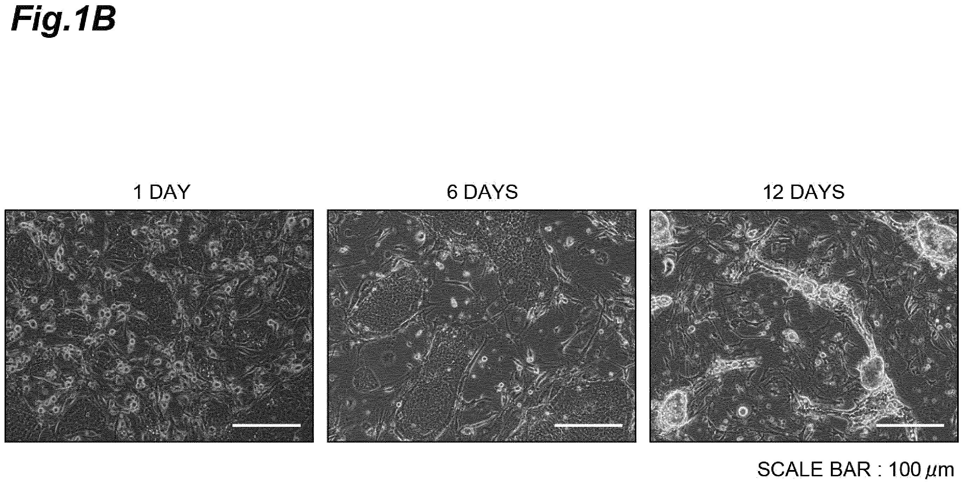

The biliary epithelial cells were cultured in the BIM-1 medium for 6 days, the medium was then exchanged for the following BIM-2 medium, and the biliary epithelial cells were further cultured for 6 days. The BIM-2 medium was newly exchanged once every 2 days during the culture. After the culture in the BIM-2 medium for 6 days, bile ducts formed from the biliary epithelial cells were observed (rightmost figure in Figure 1B).

BIM-2 medium: medium containing the BIM-1 medium and 2% Matrigel.

### <Coculture with rCLiP bile ducts and primary hepatocytes>

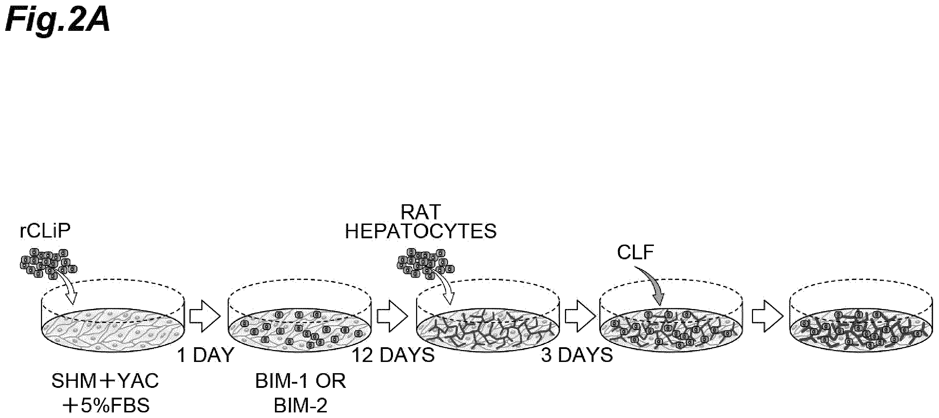

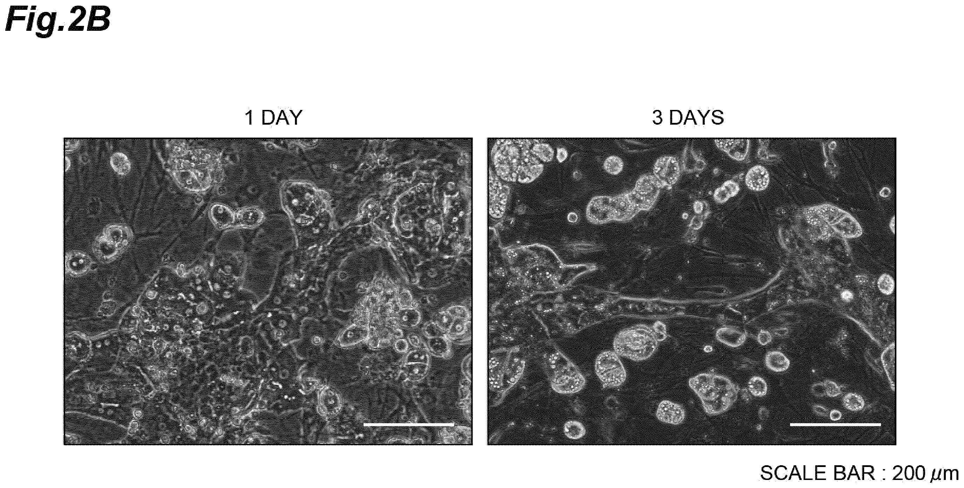

As illustrated in Figure 2A below, the bile ducts formed from the rCLiPs and primary hepatocytes were cocultured.

Rat primary hepatocytes perfused by the two-step collagenase perfusion method and separated were inoculated at a density of 5.3 × 10⁴ cells/cm² into the medium in which the biliary epithelial cells were induced from the rCLiPs as mentioned above to form bile ducts (after the culture in the BIM-1 medium for 6 days and in the BIM-2 medium for 6 days). Hepatocyte culture medium was used for the medium at the time of inoculating the primary hepatocytes.

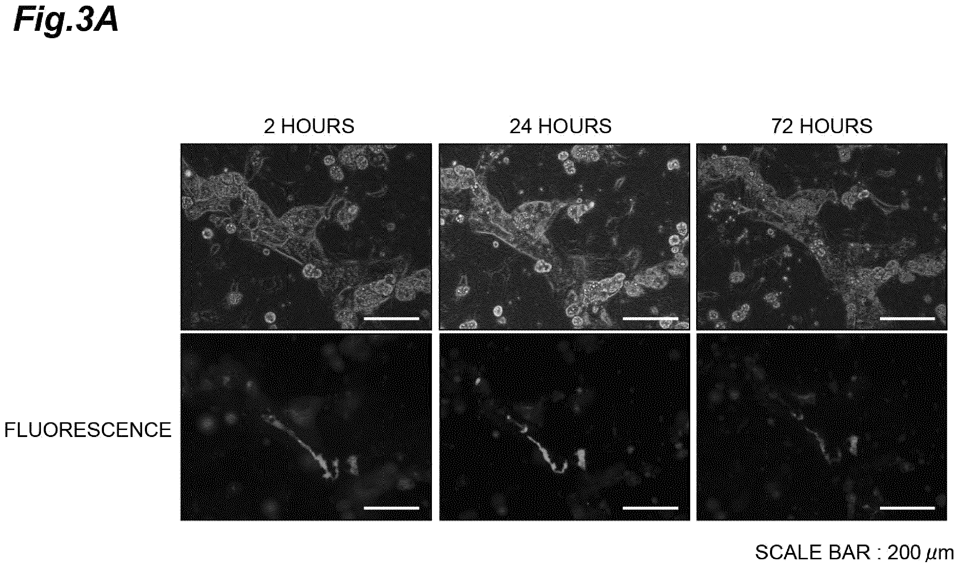

One day after the inoculation of the primary hepatocytes, the medium was exchanged for the BIM-2 medium. Three days after the inoculation of the primary hepatocytes, a CLF solution was added to the medium to evaluate the functional connection of hepatocytes-biliary canaliculi-bile ducts. The appearances of the cells 2 hours, 24 hours, and 72 hours after the addition of the CLF solution were observed through a phase-contrast microscope and a fluorescence microscope (Figure 3A). The excitation wavelength at the time of the observation through the fluorescence microscope was 480 nm.

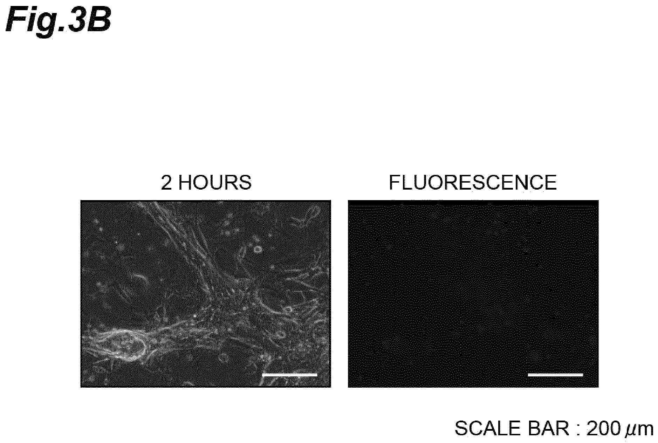

As a negative control, a CLF solution was added without the inoculation of primary hepatocytes into medium in which biliary epithelial cells were similarly induced from rCLiP to form bile ducts (after the culture in the BIM-1 medium for 6 days and in the BIM-2 medium for 6 days). The appearance 2 hours after the addition of the CLF solution was observed through the phase-contrast microscope and the fluorescence microscope (Figure 3B).

As illustrated in Figure 3A, it was shown that fluorescence was detected from the bile ducts, the CLF incorporated into the primary hepatocytes was secreted to the biliary canaliculi, and moved from the biliary canaliculi to the bile ducts. That is, in the produced cultured tissue, the biliary canaliculi and the bile ducts could be functionally connected. As illustrated in Figure 3B, fluorescence was not, meanwhile, detected even though CLF was added to the medium containing only the bile ducts.

### Example 2. Production of cultured tissue using human hepatocytes

Moreover, the present invention will be further described by Example not using rat hepatocytes but using human hepatocytes. However, the present invention is not interpreted within limitation to the following Examples.

### <Preparation of rat CLiPs>

Rat hepatocytes were reprogrammed to liver progenitor cells in accordance with the method described in the above-mentioned Reference 1.

The liver of an 8-week-old male Wistar rat was specifically perfused by the two-step collagenase perfusion method, primary hepatocytes were separated, and the primary hepatocytes were then inoculated on a collagen-coated dish at a density of 1 × 10⁴ cells/cm². The following SHM (small hepatocyte culture medium) + YAC medium was used for the medium.

SHM+YAC medium: Medium containing, in the basic medium (DMEM/F12 medium), the following: 5 mM HEPES, 30 mg/L L-proline, 0.05% BSA, a 10 ng/mL epidermal growth factor (EGF), 1% insulin-transferrin-sodium selenite (ITS)-X supplement (produced by Thermo Fisher Scientific K.K.), 10 µM dexamethasone, 10 mM nicotinamide, 1 mM ascorbic acid-2 phosphoric acid (Asc2P), 100 units/mL penicillin-100 µg/mL streptomycin-250 ng/mL amphotericin B (Antibiotic-Antimycotic), 10 µM Y-27632, 0.5 µM A-83-01, and 3 µM CHIR99021.

One day after the inoculation of the rat primary hepatocytes, the medium was exchanged for the SHM + YAC medium, the medium was then exchanged once every 2 to 3 days, culture was performed for 12 to 15 days until the cells were confluent, and rCLiPs were produced.

The rCLiPs were treated with TrypLE (TM) Express (produced by Thermo Fisher Scientific K.K.) for 10 to 15 minutes, exfoliated from the dish, and subcultured.

### <Induction from rCLiPs to biliary epithelial cells and bile duct formation>

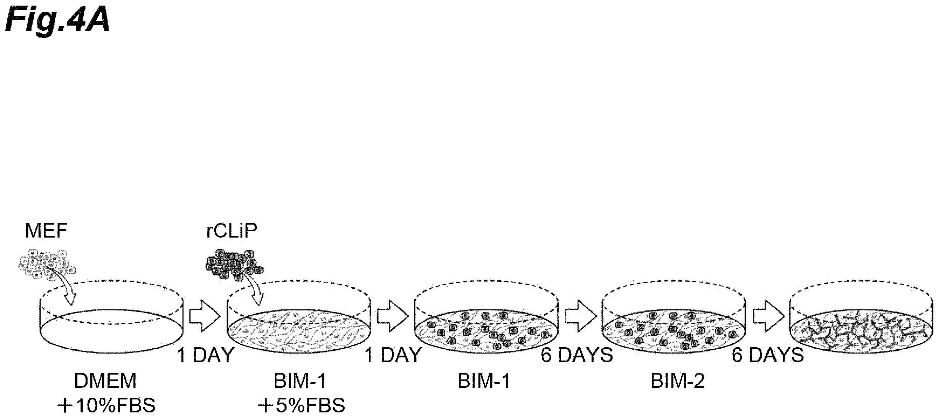

As illustrated in Figure 4A below, biliary epithelial cells were induced from rCLiPs, and bile ducts were formed.

Mitomycin-treated mouse embryonic fibroblasts (MEFs) were inoculated on a collagen-coated 12-well plate at a density of 5.3 × 10⁴ cells/cm² and cultured for 1 day. High glucose DMEM medium containing 10% fetal bovine serum (FBS) was used for the medium at the time of inoculating the MEFs.

One day after the inoculation of the MEFs, the subcultured rCLiPs were inoculated on the MEFs at a density of 1.3 × 10⁵ cells/cm². Medium obtained by adding 5% FBS to the following BIM (bile duct-inducing medium)-1 medium was used for the medium at the time of inoculating rCLiPs.

BIM-1 medium: Medium containing mTeSR(TM) 1 (produced by STEMCELL (TM) Technologies Inc.), 10 µM Y-27632, 0.5 µM A-83-01, and 3 µM CHIR99021.

One day after the inoculation of the rCLiPs, the medium was exchanged for BIM-1 medium, culture was then performed for 6 days to obtain biliary epithelial cells. The BIM-1 medium was newly exchanged once every 2 to 3 days during the culture.

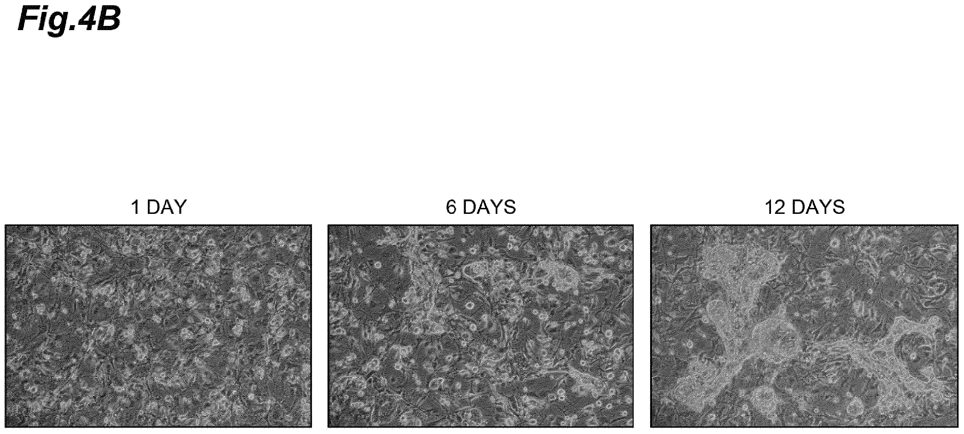

The biliary epithelial cells were cultured in the BIM-1 medium for 6 days, the medium was then exchanged for the following BIM-2 medium, and the biliary epithelial cells were further cultured for 6 days. The BIM-2 medium was newly exchanged once every 2 to 3 days during the culture. After culture in the BIM-2 medium for 6 days, bile ducts formed from the biliary epithelial cells were observed (rightmost figure in Figure 4B).

BIM-2 medium: Medium containing BIM-1 medium and 2% Matrigel.

### <Coculture of rCLiP bile ducts and human hepatocytes>

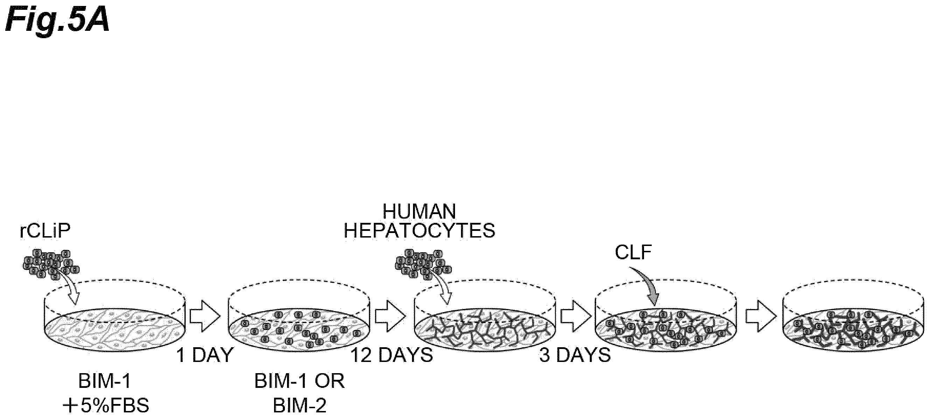

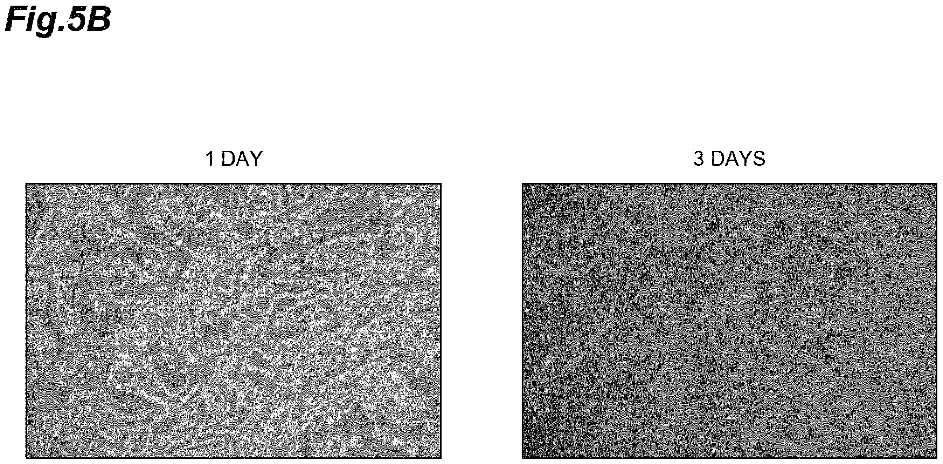

As illustrated in Figure 5A and Figure 5B below, the bile ducts formed from the rCLiPs and human hepatocytes were cocultured.

### (Preparation of human hepatocyte suspended solution based on frozen human hepatocytes)

Frozen human hepatocytes were thawed using Cryopreserved Hepatocytes Recovery Medium (CHRM (R)) (produced by Thermo Fisher Scientific K.K.), the cells were centrifuged, and the supernatant was removed. William's Medium E containing a Primary Hepatocyte Thawing and Plating Supplement (produced by Thermo Fisher Scientific K.K.) was then added to the human hepatocytes to prepare a human hepatocyte suspended solution.

The medium was removed from the culture wells of the bile ducts formed by inducing the biliary epithelial cells from the rCLiPs as mentioned above (after the culture in the BIM-1 medium for 6 days and in the BIM-2 medium for 6 days), and the above-mentioned human hepatocytes were inoculated at a density of 5.3 × 10⁴ cells/cm².

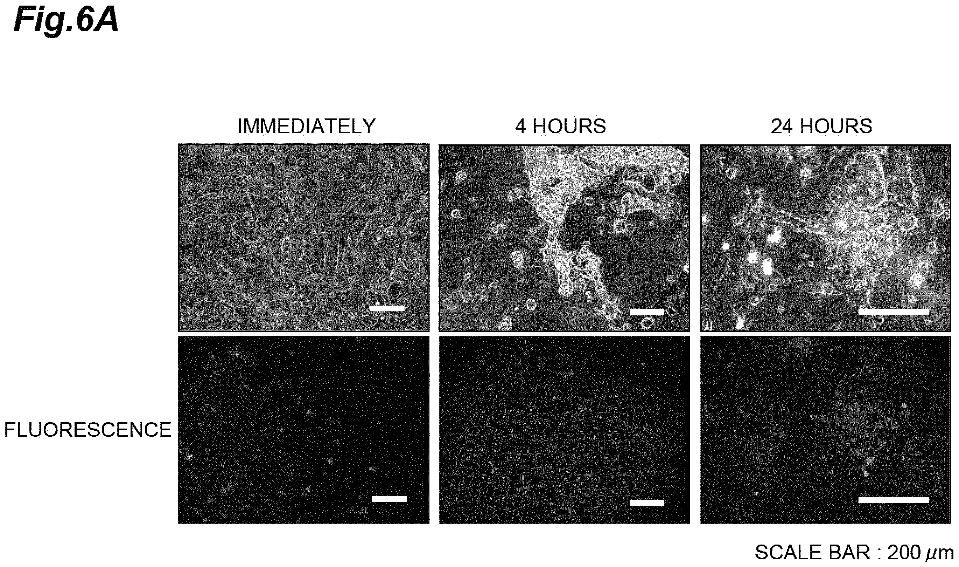

One day after the inoculation of the human hepatocytes, the medium was exchanged for BIM-2 medium. Three days after the inoculation of the human hepatocytes, the medium was exchanged for Hanks' balanced salt solution (HBSS) (produced by Thermo Fisher Scientific K.K.) containing CLF, and incubation was performed for 30 minutes to evaluate the functional connection of hepatocytes-biliary canaliculi-bile ducts. After the incubation, the cells were washed with HBSS not containing CLF, and the appearances of the cells immediately, 4 hours, and 24 hours after washing were observed through a phase-contrast microscope and a fluorescence microscope (Figure 6A). The excitation wavelength at the time of the observation through the fluorescence microscope was 470 nm.

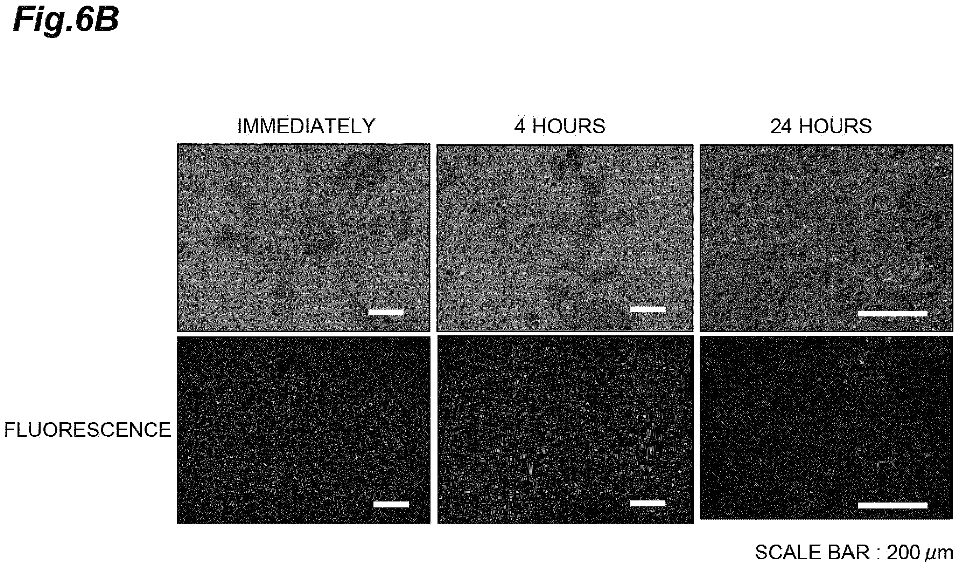

As a negative control, biliary epithelial cells were similarly induced from the rCLiPs, the medium was exchanged for HBSS containing CLF without inoculating the human hepatocytes into a well in which bile ducts were formed, and incubation was performed for 30 minutes. After the incubation, the cells were washed with HBSS not containing CLF, and the appearances of the cells immediately, 4 hours, and 24 hours after the washing were observed through a phase-contrast microscope and a fluorescence microscope (Figure 6B).

As illustrated in Figure 6A, it was shown that fluorescence was detected from the bile ducts, the CLF incorporated into the human hepatocytes was secreted to the biliary canaliculi, and moved from the biliary canaliculi to the bile ducts. That is, the biliary canaliculi and the bile ducts could be functionally connected in the produced cultured tissue. As illustrated in Figure 6B, even though the medium containing only the bile ducts was exchanged for HBSS containing CLF without inoculating the human hepatocytes, and incubation was performed, fluorescence was not, meanwhile, detected.

## Claims

1. A cultured tissue, comprising:
glandular cells;
glandular cavities formed from the glandular cells; and
ducts formed from epithelial cells,
wherein the glandular cavities and the ducts are functionally connected *ex vivo.*

2. The cultured tissue according to claim 1, wherein the cultured tissue is an exocrine gland.

3. The cultured tissue according to claim 1 or 2, wherein the glandular cells are hepatocytes, and the epithelial cells are biliary epithelial cells.

4. The cultured tissue according to claim 1 or 2, wherein the glandular cells are pancreatic acinar cells, and the epithelial cells are pancreatic ductal epithelial cells.

5. The cultured tissue according to any one of claims 1 to 4, wherein the epithelial cells are epithelial cells induced from progenitor cells.

6. The cultured tissue according to claim 5, wherein the progenitor cells are progenitor cells reprogrammed from mature cells.

7. The cultured tissue according to any one of claims 1 to 6, wherein the glandular cells are primary cells.

8. The cultured tissue according to claim 3, wherein the glandular cells are primary hepatocytes, and the biliary epithelial cells are biliary epithelial cells induced from liver progenitor cells, and wherein the liver progenitor cells are liver progenitor cells reprogrammed from hepatocytes.

9. The cultured tissue according to any one of claims 1 to 8, wherein the epithelial cells are rodent cells.

10. The cultured tissue according to any one of claims 1 to 9, wherein the glandular cells are human cells.

11. The cultured tissue according to any one of claims 1 to 3 and 5 to 10, wherein the glandular cells are human hepatocytes.

12. The cultured tissue according to any one of claims 1 to 3 and 5 to 11, wherein the epithelial cells are rodent biliary epithelial cells.

13. A method for producing a cultured tissue, comprising:
culturing epithelial cells to form ducts from the epithelial cells; and
coculturing the ducts and glandular cells so that the ducts and the glandular cells are in contact.

14. The method according to claim 13, wherein the coculture is performed by two-dimensional culture.

15. The method according to claim 13 or 14, wherein the glandular cells are hepatocytes, and the epithelial cells are biliary epithelial cells.

16. The method according to claim 13 or 14, wherein the glandular cells are pancreatic acinar cells, and the epithelial cells are pancreatic ductal epithelial cells.

17. The method according to any one of claims 13 to 16, further comprising:
inducing differentiation of progenitor cells to the epithelial cells.

18. The method according to claim 17, further comprising:
reprogramming mature cells to the progenitor cells before the induction of differentiation of the progenitor cells to the epithelial cells.

19. The method according to any one of claims 13 to 18, wherein the glandular cells are primary cells.

20. The method according to any one of claims 13 to 19, wherein the epithelial cells are rodent cells.

21. The method according to any one of claims 13 to 20, wherein the glandular cells are human cells.

22. The method according to any one of claims 13 to 15 and 17 to 21, wherein the glandular cells are human hepatocytes.

23. The method according to any one of claims 13 to 15 and 17 to 22, wherein the epithelial cells are rodent biliary epithelial cells.

24. A method for evaluating a test substance using the cultured tissue according to any one of claims 1 to 12.

25. A kit for evaluating a test substance, comprising the cultured tissue according to any one of claims 1 to 12.
